# EUROPEAN PATENT APPLICATION

(11) **EP 3 991 762 A1**
(43) Date of publication of application: **04.05.2022**
(21) Application number: 20205219.7
(22) Date of filing: 02.11.2020
(51) Int. Cl.: A61L 27/22

(54) **IMPLANT FOR NERVE REGENERATION**

(71) Applicant: Medizinische Hochschule Hannover, 30625 Hannover (DE)
(72) Inventor: VOGT, Peter, 30625 Hannover (DE); LIEBSCH, Christina, 30625 Hannover (DE); BUCAN, Vesna, 30625 Hannover (DE); HAVERICH, Axel, 30625 Hannover (DE); STRAUSS, Sarah, 30625 Hannover (DE); APER, Thomas, 30625 Hannover (DE); WILHELMI, Mathias, 30625 Hannover (DE)
(74) Representative: Taruttis, Stefan Georg

(57) **Abstract**

The present invention relates to an implant suitable for nerve regeneration, also referred to as a neural implant, that is especially suitable for bridging defect sites of nerves, especially defect sites within the central nervous system, e.g. for spanning or bridging defects of axons within the spinal cord, which implant comprises or consists of a sheath, which preferably about its axis is circumferentially closed between its ends, the sheath consisting of fibrin and silk fibers arranged in axial direction inside the sheath, optionally fibrin that fills the inner volume of the sheath, e.g. fibrin filling the inner volume of the sheath and between the silk fibers.

## Description

The present invention relates to an implant suitable for nerve regeneration, also referred to as a neural implant. The implant contains silk fibers arranged longitudinally in a sheath, which provides for mechanical stability suitable for handling the implant, e.g. during surgery, and suitable for connecting the implant to tissue, e.g. connecting its ends to nerve stumps. The implant is especially suitable for bridging defect sites of nerves, especially defect sites within the central nervous system, e.g. for spanning or bridging defects of axons within the spinal cord. It was found that the implant in a recipient allows the migration and growth of neurites, e.g. of dorsal root ganglia, along the silk fibers of the implant. Defect sites of nerves are e.g. interruptions or gaps within a nerve.

Further, the invention provides a process for producing the implant, which has the advantage of not comprising any steps comprising mammalian cells, e.g. the process is devoid of cultivating cells or integrating cells into the implant, and preferably the process is devoid of any cell growth factors or hormones, e.g. the process preferably does not involve integrating any cell growth factors or hormones into the implant.

In a preferred embodiment, the implant has a sheath that consists of polymerized natural compounds and the process for producing the sheath of the implant only uses natural compounds, e.g. polymerization of only natural compounds.

Further, the invention provides a method for bridging defects of nerves, especially bridging defects of axons within the spinal cord, by implanting the implant of the invention into the site of the defect and arranging the nerve stumps that border the defect site adjacent to the implant, preferably adjacent to the end cross-sections of the implant. Preferably, the nerve stumps that delimit the defect of nerves are connected to the implant, especially connected to the silk fibers arranged at the terminal cross-sections of the implant. Nerve stumps can be connected to the silk fibers of the implant by gluing, e.g. using fibrin glue, and/or by sewing. The defect site of a nerve can be a recent defect, e.g. caused by mechanical interruption of the nerve, e.g. within 2 h up to 2 d prior to inserting the implant into the defect site, or the defect site can be an old mechanical interruption of the nerve, e.g. at least 2 days or more prior to inserting the implant. It was found that especially for defects of nerves like interruptions of the spinal cord, the implant and the method of the invention are suitable also for use in treatment of old defects.

### State of the art

WO 2017/005857 A1 describes a process for producing a tube of high density fibrin that is suitable as an implant and as a matrix for a blood vessel prosthesis.

WO2013/091865 A1 describes a process for producing a tissue construct that contains living mammalian cells by casting cells and fibrinogen in a rotating tubular mould.

WO2008/140416 A1 describes a process for producing a fibrin-based nerve repair conduit by curing a tissue glue forming fibrinogen and serin protease in a mould having a central shaft.

Wang et al., The Anatomical Record 301: 1690-1696 (2018) describe fibrin conduits consisting of fibrin obtained by statically pressing two-compound fibrin glue in a silicone mould, and after polymerization using the conduits as implants for repairing nerve defects.

WO2007/028801 A2 describes a neural implant of spider silk that can consist of braided spider silk or of spider silk arranged in an acellularized venule.

US 8 106 014 B2 describes a neural implant of silkworm silk fibers arranged in a biodegradable tube.

US2005/0260706 A1 describes a tissue engineered construct comprising electrospun fibers of silk protein, which preferably is fibroin from dissolved silkworm silk, in a mixture with a polymer, e.g. polyethylene oxide, PEG, collagen, fibronectin, keratin, polyaspartic acid, polylysine, alginate, chitosan, chitin, hyaluronic acid, pectin, polycaprolactone, polylactic acid, polyglycolic acid, polyhydroy alkanoates, dextrans, and polyanhydrides.

EP2097117 B1 describes an implant suitable as a scaffolding matrix which is produced by moulding and cross-linking spider silk threads.

### Object of the invention

It is an object of the invention to provide an alternative neural implant, preferably a neural implant suitable for bridging Preferably, the neural implant has a structure that promotes nerve growth, e.g. along the implant. More preferably, the neural implant shall be easy to handle and shall have a mechanical stability that allows connecting it with nerve stumps in a recipient, and the neural implant shall be accessible by a simple production method.

### Description of the invention

The invention achieves the object by the features of the claims, and especially by an implant that is especially suitable for bridging defect sites of nerves, especially defect sites within the central nervous system, e.g. for spanning or bridging defects of axons within the spinal cord, which implant comprises or consists of
a sheath, which preferably about its axis is circumferentially closed between its ends, the sheath consisting of fibrin and
silk fibers arranged in axial direction inside the sheath,
optionally fibrin that fills the inner volume of the sheath, e.g. fibrin filling the inner volume of the sheath and between the silk fibers.

The ends of the sheath can form an open cross-section that is enclosed by the sheath, and optionally the inner volume of the sheath and between the silk fibers can be filled with fibrin, e.g. up to the cross-section of the sheath.

The sheath preferably is circumferentially closed, e.g. by the sheath being continuous and having a closed surface. Alternatively, the sheath can be in the form of a strip-shape that is arranged, e.g. wound around the silk fibers, wherein preferably the long edges of the strip-shape overlap. A sheath in the form of a strip-shape can be produced by first producing a circumferentially closed sheath by polymerizing fibrinogen to fibrin in a rotating mould, removing the circumferentially closed sheath from the mould and cutting the circumferentially closed sheath into a strip-shape, e.g. cutting lengthwise or spirally, e.g. after inserting a support, e.g. a Teflon-coated round steel rod, into the circumferentially closed sheath.

The process of the invention for producing the implant comprises or consists of producing a sheath consisting of fibrin, the sheath extending along a longitudinal axis, preferably drying the sheath before or after arranging silk fibers in the sheath to generate a dried sheath, and, before or subsequent to arranging silk fibers in the sheath, re-hydrating the dried sheath to generate a re-hydrated sheath,
arranging silk fibers in the sheath, the fibers extending along the longitudinal axis of the sheath,
and preferably filling the inner volume of the sheath and between the silk fibers with fibrin, e.g. by introducing fibrinogen and, concurrently or subsequently, thrombin into the inner sheath volume.

Preferably, the sheath consists of fibrin having a high density, e.g. obtainable by polymerizing a fibrinogen containing solution in a rotating mould, which preferably about its longitudinal axis is rotationally symmetrical and has a constant diameter and rotates to generate a g-force of at least 200 x g, e.g. at least or up to 900 x g, preferably 300 x g to 500 x g, e.g. 400 x g, the g-force being determined at the inner wall of the rotating mould.

Optionally, olfactory ensheathing cells (OEC) are introduced into the fibrin sheath, e.g. embedded in the fibrin filling the inner volume of the fibrin sheath. Olfactory ensheathing cells (OEC) can e.g. be autologous cells derived from the later recipient of the implant, and OEC can be increased in number by cultivating OEC derived from the later recipient of the implant prior to introducing the OEC into the sheath.

Optionally, the silk fibers can be arranged in the sheath while the sheath has a state of hydration that originates from its production.

Preferably, an insert, e.g. of plastics or steel, is inserted into the fibrin sheath prior to drying, and drying the fibrin sheath with the insert inserted, before or after arranging silk fibers in the fibrin sheath to generate a dried fibrin sheath, and, before or subsequent to arranging silk fibers in the fibrin sheath, re-hydrating the dried fibrin sheath to generate a re-hydrated fibrin sheath, optionally removing the insert from the dried fibrin sheath before or after re-hydrating. The insert that is inserted into the fibrin sheath prior to drying has the advantage of determining the inner diameter of the dried fibrin sheath, and it has been found that an insert of plastics or of steel can easily be withdrawn from the sheath prior to or after re-hydrating the dried fibrin sheath. Further, the insert present in the fibrin sheath during the drying preferably also determines the inner diameter of the fibrin sheath after re-hydrating it, e.g. preferably determines the inner diameter of the re-hydrated fibrin sheath to be the outer diameter of the insert.

The mould preferably has a smooth inner surface, e.g. of steel, preferably of poly tetrafluoro ethylene (available as Teflon), and the mould has drainage bore holes for draining water and solubles which are not polymerized into fibrin. The bore holes can be distributed about the circumference and length of the rotating mould, e.g. every 5 to 20 mm of the length and two or more bore holes radially offset at one axial section of the mould. The bore holes can have diameters e.g. between 0.05 and 0.8 mm, e.g. between 0.1 and 0.6 or 0.5 or 0.4 mm. The rotating mould can e.g. have an inner diameter of 4 to 15 mm, e.g. 5 to 10 mm, preferably of 6 mm or 7 mm to 8 mm. Preferably, the outside surface of the mould is covered with a semipermeable membrane that retains fibrin but allows water to pass. The end sections of the mould preferably have a smaller inner diameter, e.g. projecting over the inner surface of the mould axially towards the longitudinal axis of the mould, for retaining solution on the inner surface of the rotating mould to a thickness corresponding to the end sections projecting over the inner surface of the rotating mould. The end sections can e.g. project over the inner surface of the rotating mould by 1.5 to 2.5 mm, e.g. for 2 mm, and optionally the end sections can have a central opening allowing aqueous and dissolved compounds to leave the mould.

The sheath of fibrin that is produced by polymerizing a fibrinogen containing solution to fibrin in a rotating mould preferably has a tensile strength of at least 12 to 50 kPa, e.g. 28 ± 10 kPa and has an elastic modulus of 2.8 ± 1.3 kPa. The tensile strength is measured along the longitudinal axis of the fibrin sheath, e.g. for a circumferentially closed fibrin sheath, and e.g. along the longitudinal axis of a strip forming a fibrin sheath by being wound around silk fibres.

It has been found that the fibrin sheath, which is produced by the process, for growth of nerve cells preferably has pores between fibrin fibrils of 13 to 80 µm, e.g. of 35 ± 23 µm. These pore sizes have been found to favour the growth of nerve cells.

The silk fibers can terminate in the cross-section of the sheath, or the silk fibers can project out of the cross-section of the sheath, e.g. extend beyond the sheath. The ends of the silk fibers can be blunt ends, e.g. cut ends. Alternatively, the ends of the silk fibers can be loops, e.g. formed by a continuous loop between portions of silk fibers arranged inside and along the longitudinal axis of the sheath.

The implant of the invention has the advantage of being producible without containing cells, and accordingly the implant is devoid of cells, and can be stored in a dry state or in a hydrated state. Optionally, the implant is devoid of growth factors. Preferably, the implant is generated from autologous or allogenic plasma or whole blood, or from commercially available fibrinogen preparations, e.g. available as Haemocomplettan P from CSL Behring, Germany.

The fibrin that optionally fills the inner volume of the sheath and between the silk fibers is polymerized from a fibrinogen containing solution in the absence of pressure or g-force, but is allowed to polymerize under ambient pressure, e.g. under static conditions. This fibrin filling can have a tensile strength of 3.7 ± 1.7 kPa, and an elastic modulus of 0.06 ± 0.01 kPa, with interstices between fibrin fibrils of e.g. 140 to 220 µm, e.g. 160 ± 30 µm.

Subsequent to the production of the sheath by polymerizing fibrinogen from a solution in a rotating mould, the sheath can optionally be dried and, prior to or subsequent to arranging silk fibers in the sheath, be rehydrated. Drying of the sheath subsequent to producing it in the rotating mould is preferred, as it has been found that the drying increases the density of the fibrin sheath, and respectively reduces pores in the sheath, e.g. to pore sizes of 35 ± 23 µm.

Alternatively, silk fibers can be arranged in the sheath without prior drying of the sheath but essentially maintaining the water content of the sheath generated by the production of the sheath in the rotating mould.

Silk fibers can be arranged in the sheath after the sheath coming out of the mould has been dried, e.g. in a desiccator over dry NaCl or at 25% relative humidity at room temperature up to constant weight.

Herein, the silk fibers preferably are natural spider silk fibers or fibers spun from natural compounds only. Natural spider silk fibers are e.g. the major ampullate silk fiber, preferably obtained from spiders of the Nephila genus, e.g. Nephila clavipes. Silk fibers spun from natural compounds are silk fibers that are produced from natural compounds only, which are artificially spun, e.g. electro-spun, e.g. from dissolved silk protein or silk protein starting compounds, e.g. dissolved spider silk, dissolved silkworm silk, or dissolved silk obtained from insects, e.g. moths or butterflies, or mixtures of at least two of these as starting material. Therein, the dissolved silk protein can be protein having the amino acid sequence of a natural silk fiber, which protein is expressed in a micro-organism, e.g. expressed in bacteria, yeast or fungal cells, or cultivated mammalian cells, each genetically manipulated to express silk protein.

Herein, natural compounds include or consist of chemical compounds that are isolated from a natural source and chemical compounds that are obtained by chemical processes and are chemically identical to the compounds isolated from a natural source.

The invention is now described in greater detail by way of an example.

### Example: Production of the implant for bridging a gap in a nerve

A sheath consisting of fibrin was produced by introducing into a mould an aqueous mixture of fibrinogen solution, e.g. available as Haemocomplettan from CSL Behring or obtained by cryoprecipitation and separation from human plasma or whole blood, concurrently with thrombin as the activator for polymerization to fibrin. The mould was a 10 cm long metal tube, inner diameter 9 mm, with a total of 14 drainage bore holes, 0.5 mm diameter, distributed pairwise and opposite over the circumference, with a Teflon inset of two half-moulds forming a poly tetrafluoro ethylene coating of the inner mould surface to an inner diameter of 7 mm. The inner mould surface is cylindrical about a longitudinal axis. The end sections of the mould received inserts that reduced the inner diameter of end sections of the mould to 3 mm to prevent the fibrinogen solution from exiting the mould. The fibrinogen containing mixture was generated by concurrently introducing 5 mL aqueous fibrinogen solution at a flow rate of 120 mL/h having a concentration of 20 to 30 mg/mL fibrinogen, and an aqueous thrombin solution containing 40 mM CaCl₂, 1000 units aprotinin (obtained from Loxo), and 100 units thrombin (obtained from Baxter) at a flow rate of 80 mL/h, each to coupling leading to one common tubing that discharged into the mould while the mould was rotated at 5500 rpm, resulting in a centrifugal force about 120 x g. The common tube was connected to a 0.9 x 40 mm canula that was introduced axially in the mould and was retracted continuously from the mould during the discharge of the mixture of the fibrinogen solution and the thrombin solution. The mould was rotated about its longitudinal axis at about 10 000 rpm, resulting in a g-force of about 400 x g on the inner surface of the mould, for about 10 min. This process was preferably carried out at room temperature.

Subsequently, the polymerized sheath consisting of fibrin was removed from the mould. Prior to drying, i.e. directly after removal from the mould, the fibrin sheath had a maximum tensile strength of 10 to 17 kPa, e.g. 12 ± 2 kPa, and an elastic modulus of 0.8 to 1.5 kPa, e.g. 1.1 ± 0.3 kPa.

Subsequently, the fibrin sheath was dried over NaCl at room temperature. Preferably, prior to drying, an insert, e.g. of plastic or of steel, was inserted into the fibrin sheath to generate a dried sheath. The insert prevented a collapse of the fibrin sheath and it was found that the insert determined the inner diameter of the dried fibrin sheath. The dried sheath was re-hydrated in aqueous 40 mM CaCl₂, optionally containing 1 % vol/vol antibiotic solution (available as CellCultureGuard from PanReac Applichem, Germany), and containing 1000 units /mL aprotinin. This re-hydration solution is also preferred for storing the implant. Generally preferred, the water used in the process is aqua ad iniectabilia. Re-hydration was for 1 h at 4 °C, then the insert could be retracted, and the fibrin sheath could be stored in this solution at 4 °C. After the drying and re-hydration, the fibrin sheath had a maximum wall tension, also referred to as tensile strength, that was increased in comparison to a fibrin sheath directly after polymerization in the mould and without drying. In this example a maximum wall strength (tensile strength) of 12.4 to 46.1 kPa was determined for the dried and re-hydrated fibrin sheaths from several repetitions. The pores between fibrin fibrils of the dried and re-hydrated fibrin sheath from several repetitions were determined to have a diameter of 13-80 µm.

As the preferred example for silk fibers, natural spider silk, namely the major ampullate fiber of Nephila clavipes, was carefully inserted into a 4 mm long section of the fibrin sheath until 50 to 200 fibers were arranged longitudinally in the sheath. Subsequently, the sheath was filled with a mixture of 20 mg/mL fibrinogen in water, admixed with 10 units thrombin in 40 mM CaCl₂ This fibrin filling the inner volume of the fibrin sheath and enclosing the silk fibers within the sheath had a maximum tensile strength of 3.7 ± 1.7 kPa and an elastic modulus of 0.06 ± 0.01 kPa, with pores between fibrin fibres of 164 ± 31 µm.

This implant could be store in the storage solution containing aqueous 40 mM CaCl₂, optionally containing 1 % vol/vol antibiotics solution (available as CellCultureGuard), and containing 1000 units /mL aprotinin, at 4 °C without apparent deterioration of its properties.

The implant having a length of 3 to 4 mm was surgically inserted into a gap that was cut in the spinal cord of a rat, obeying animal care provisions for experimental animals. It was found that the implant resulted in the ingrowth of nerve cells, e.g. of dorsal root ganglia, and restoration of the nerve tissue.

In an in vitro assay, a section of the implant was incubated for 22 days in cultivated olfactory ensheathing cells (OEC) under static cell culture conditions. Analysis of the implants showed that the OEC migrate and proliferate on the spider silk fibers within the fibrin sheath. The proportion of living cells inside the implant was more than 98%, with no signs for toxicity detected.

As a further in vitro assay, live sections of rat spinal cord tissue were placed on top of implants, preferably on top of the cross-sectional end, horizontally arranged, of an implant and then cultivated under cell culture conditions. Analyses by immune fluorescence staining showed that neurons of the central nervous system as well as astrocytes, oligodendrocytes and microgliacells were found on silk fibers of the implant. It is assumed that these cells have migrated or grown out of the spinal cord tissue directly into the implant and along the silk fibers. Scanning electron microscopic pictures show that the nerve cells are oriented along the silk fibers.

## Claims

1. Implant for nerve regeneration, comprising a sheath consisting of fibrin, comprising silk fibers arranged in axial direction inside the sheath, wherein the inner volume of the sheath and between the silk fibers is filled with fibrin.

2. Implant according to claim 1, **characterized in that** the sheath is circumferentially closed.

3. Implant according to claim 1, **characterized in that** the sheath is a strip that is wound around the silk fibers, wherein the long edges of the strip overlap.

4. Implant according to one of the preceding claims, **characterized in that** the sheath has pores between fibrin fibrils of 13 to 80 µm and/or the sheath has tensile strength of at least 12 to 50 kPa.

5. Implant according to one of the preceding claims, **characterized in that** the sheath consists of high density fibrin that produced by polymerizing fibrinogen to fibrin in a rotationally symmetrical mould rotating about its longitudinal axis generating a g-force at its inner surface of at least 200 x g.

6. Implant according to one of the preceding claims, **characterized in that** the sheath is produced by polymerizing fibrinogen to fibrin, followed by drying the sheath before or after arranging silk fibers in the sheath to generate a dried sheath, and subsequently re-hydrating the sheath.

7. Implant according to one of the preceding claims, **characterized in that** the silk fibers are selected from spider silk fibers obtained from spiders, fibers spun from dissolved silk protein or silk protein starting compounds.

8. Implant according to one of the preceding claims, **characterized in that** the silk fibers project over the ends of the sheath and the silk fibers have cut ends or the silk fibers form a continuous loop between portions of silk fibers arranged inside and along the longitudinal axis of the sheath.

9. Implant according to one of the preceding claims, **characterized in that** it consists of the sheath, the silk fibers, and fibrin filling the sheath.

10. Process for producing an implant, especially an implant according to one of the preceding claims, comprising producing a sheath consisting of fibrin by polymerizing fibrinogen to fibrin in a mould rotating to generate a g-force of at least 100 x g at its inner surface, the sheath extending along a longitudinal axis, arranging silk fibers in the sheath to extend longitudinally along the sheath, and filling the inner volume of the sheath with fibrinogen and polymerizing the fibrinogen to fibrin.

11. Process according to claim 10, **characterized by** inserting an insert into the sheath and drying the sheath before or after arranging silk fibers in the sheath to generate a dried sheath, and, before or subsequent to arranging silk fibers in the sheath, re-hydrating the dried sheath to generate a re-hydrated sheath, optionally removing the insert from the sheath before or after re-hydrating.

12. Process according to one of claims 10 to 11, **characterized in that** the sheath is produced by polymerizing fibrinogen to fibrin, followed by drying the sheath before or after arranging silk fibers in the sheath to generate a dried sheath, before or after drying arranging silk fibers in the sheath, and subsequently re-hydrating the sheath and filling the inner volume of the sheath containing silk fibers with fibrinogen to produce fibrin filling the inner volume.

13. Process according to one of claims 10 to 12, **characterized in that** the sheath is circumferentially closed.

14. Process according to one of claims 10 to 12, **characterized in that** subsequent to polymerizing, the sheath is cut into a strip to generate a strip-shape sheath, and the silk fibers are arranged in the strip-shape sheath by winding the strip-shape sheath around longitudinally extending silk fibers, wherein the long edges of the strip-shape sheath overlap.

15. Process according to one of claims 10 to 14, **characterized in that** the implant comprising or consisting of the sheath, the silk fibers, and fibrin filling the sheath is hydrated and stored at above 0 °C and below 10 °C.
